Europäisches Patentamt

**European Patent Office**

**Office européen des brevets**

(11) Veröffentlichungsnummer: **0 292 602**
A1

(12) **EUROPÄISCHE PATENTANMELDUNG**

(21) Anmeldenummer: **87111516.8**

(22) Anmeldetag: **08.08.87**

(51) Int. Cl.⁴: **A 61 B 1/00**

(30) Priorität: **26.05.87 DE 3717739**

(43) Veröffentlichungstag der Anmeldung: **30.11.88**
**Patentblatt 88/48**

(84) Benannte Vertragsstaaten: **AT BE CH DE ES FR GB GR
IT LI NL SE**

(71) Anmelder: **Storz, KarlDr.med. h.c., Auf dem
Schildrain 39, D-7200 Tuttlingen (DE)**

(72) Erfinder: **Der Erfinder hat auf seine Nennung verzichtet**

(74) Vertreter: **Wenzel, Joachim, Dipl.-Ing.,
Hauptmannsreute 46, D-7000 Stuttgart 1 (DE)**

(54) **Endoskop-Optik.**

(57)   Die Erfindung betrifft eine Endoskop-Optik mit einem Außenrohr (2) und einem die beiden Teile aus Glasmaterial wie
Linsen oder Prismen (3–7) aufnehmenden Innenrohr (1).

Um hierbei zu verhindern, daß die Linsen, Prismen oder
dergleichen aus Glasmaterial im Laufe der Zeit bei medizinischer Verwendung leiden, so daß ihre Leistungsfähigkeit gemindert ist, schlägt die Erfindung vor, daß das Außenrohr und
das Innenrohr aus unterschiedlichem Material bestehen, wobei zumindest eines ein wärmedämmendes Kunststoff-Material aufweist.

Dadurch wird verhindert, daß die hohen Sterilisationstemperaturen, der diese Instrumente ausgesetzt werden, auch im
Laufe der Zeit den Instrumenten nicht schaden.

Die Erfindung wird in erster Linie auf dem Medizin-Sektor
angewendet.

EP 0 292 602 A1

ACTORUM AG

00292602

— 1 —

Dr. med. h. c. Karl Storz, Tuttlingen


Endoskop-Optik


Die Erfindung bezieht sich auf eine Endoskop-Optik mit einem Außenrohr und einem die optischen Teile aus Glasmaterial wie Linsen oder Prismen aufnehmenden Innenrohr.


Es sind bereits derartige Endoskop-Optiken bekannt, bei denen sowohl das Außenrohr als auch das Innenrohr aus Metall, vorzugsweise Stahl besteht.


Insbesondere dann, wenn die bekannten Stablinsen im Medizin-Sektor Verwendung finden, zeigen diese Endoskop-Optiken, wenn sie neu sind, ein sehr klares Bild und weisen auch einen großen Öffnungswinkel auf. Es hat sich jedoch herausgestellt, daß die Linsen, Prismen und dergleichen/Glasmaterial im Laufe der Zeit bei dieser medizinischen Verwendung leiden, so daß ihre Leistungsfähigkeit gemindert wird.


Die Ursache hierfür war lange Zeit unbekannt, doch hat sich nun herausgestellt, daß die Ursache hierfür die hohen Sterilisationstemperaturen sind. Diese medizinischen Optiken müssen nämlich nach einer jeden Benutzung unter hohen Temperaturen sterilisiert werden.


...

0292602

- 2-

Dabei wird eine Temperatur von 140° - 150 ° C auch auf die Linsen und das sonstige Glasmaterial übertragen, wodurch die Verkittung der Linsen leidet. Selbst dann, wenn hitzefeste Kitte verwendet werden, leiden auch die Eigenschaften des Glasmaterials nach einiger Zeit, und ein Nachlassen der Bildqualität ist die Regel.

Die Erfindung geht daher von der Erkenntnis aus, daß die erwähnten Nachteile auf die erwähnten hohen Sterilisationstemperaturen zurückzuführen sind, die bei medizinischen Endoskop-Optiken unvermeidbar sind, weil die Sterilisation der Instrumente unter derart hohen Temperaturen ein Erfordernis der Medizintechnik ist.

Der Erfindung liegt die Aufgabe zugrunde, die Endoskop-Optik der eingangs erwähnten Art so zu verbessern, daß die Linsen bzw. das Glasmaterial gegen diese übermäßige Wärmeeinwirkung geschützt werden.

Zur Lösung dieser Aufgabe ist vorgesehen, daß das Außenrohr und das Innenrohr aus unterschiedlichem Material bestehen, wobei zumindest eine der beiden ein wärmedämmendes Kunststoff-Material aufweist.

Dadurch entsteht die überraschende Wirkung, daß die bisherigen Verschleißerscheinungen, die zu einer geminderten Lebensdauer

...

- 3 -

der Instrumente geführt hatten, behoben sind.

Das ist darauf zurückzuführen, daß die Linsen oder das übrige Glasmaterial keinen so hohen Temperaturen mehr  unterworfen werden,weil die hohen Temperaturen durch das wärmedämmende Kunststoffmaterial absorbiert werden.

In weiterer Ausgestaltung der Erfindung ist vorgesehen, daß im Bereich der Stellen, wo die optischen Teile aus Glasmaterial angeordnet sind, ein Spiel zwischen dem Außenrohr und dem Innenrohr vorgesehen ist. Auf diese Weise können sich Wärmebrücken nur an den Stellen ausbilden, an denen die optischen Teile aus Glasmaterial nicht vorhanden sind. Selbst dann, wenn das Spiel nur äußerst gering ist, kann sich eine Wärmebrücke nicht ausbilden.

Weitere Ausgestaltungen der Erfindungen ergeben sich aus den weiteren Unteransprüchen.

Weitere Vorteile und Einzelheiten der Erfindung ergeben sich aus der nur folgenden Beschreibung zweier Ausführungsbeispiele unter Hinweis auf die Zeichnung. In dieser zeigen:

- 4 -

Fig. 1 einen schematischen Längsschnitt durch die erste Ausführungsform und

Fig. 2 einen Längsschnitt wie Fig. 1 mit der zweiten Ausführungsform.

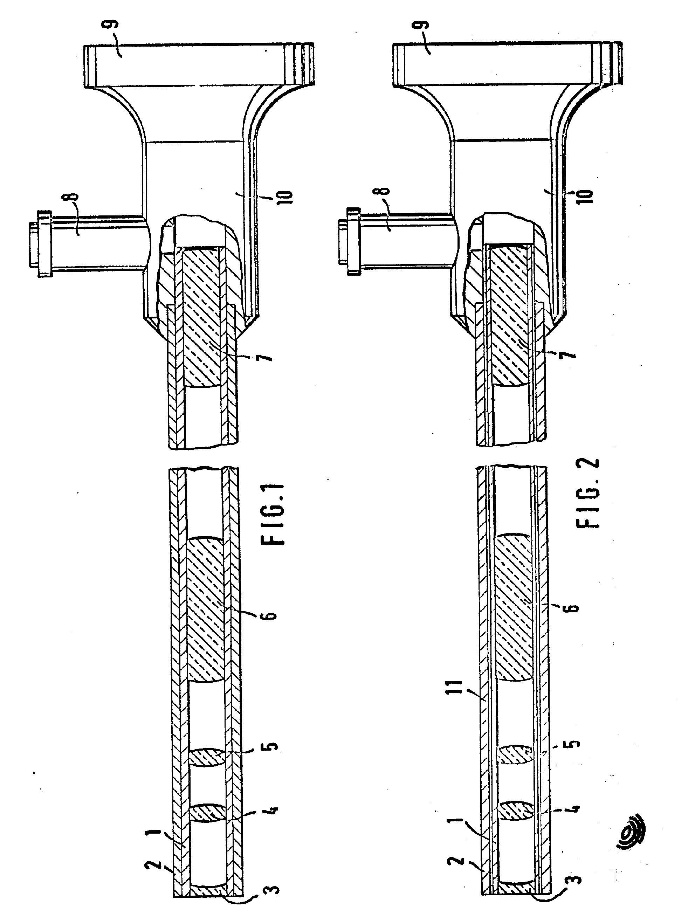

Die beiden Figuren zeigen eine Optik, die in den Endoskopschaft
eines Endoskopes in bekannter Weise eingeführt werden kann, was
dem Fachmann nicht näher erläutert werden muß. Gemäß der Fig. 1 ist
das Außenrohr 2 in dem Endoskopschaft 10 rechts befestigt, während
das Innenrohr 1 bis zum Okular 9 reicht und dazu dient, die optischen Teile aus Glasmaterial 4-7 aufzunehmen. Dabei handelt es sich,
wie ersichtlich, teilweise um Stablinsen 6, 7, die dem Fachmann
ebenfalls nicht näher erläutert werden müssen. Oben rechts am
Kopf 10 sieht man den Anschluß 8 für den Lichtleiter, der hier
nicht dargestellt ist, weil dies nach dem Stand der Technik ebenfalls
bestens bekannt ist.

Gemäß der Erfindung ist nun zunächst vorgesehen, daß das Außenrohr 2
und das Innenrohr 1 aus unterschiedlichem Material bestehen, wobei zumindest eines der beiden ein wärmedämmendes Kunststoff-Material aufweist.

Dies bedeutet, daß eines der beiden Rohre 1, 2 ein solches dem Fachmann bestens bekanntes Kunststoff-Material aufweist.

- 5 -

Derartige wärmedämmende Materialien sind dem Fachmann ebenfalls bestens bekannt, so daß das nicht im einzelnen genannt werden muß.

Zusätzlich besteht durch die Erfindung nun noch die nun folgende Möglichkeit:

An den Stellen des Innenrohres 1, wo die optischen Teile 4-7 angeordnet sind, ist ein Spiel zwischen dem Innenrohr 1 und dem Außenrohr 2 vorgesehen. Dadurch können sich an diesen Stellen keine kurzen Wärmebrücken zwischen dem Außenrohr und dem Innenrohr bilden, durch die das Glasmaterial überhitzt werden könnte.

Nach einer weiteren Ausgestaltung der Erfindung besteht die Möglichkeit, daß ein Kunststoff-Belag auf das Innenrohr 1 oder das Außenrohr 2 aufgebracht wird. Dieser Belag kann verhältnismäßig dünn sein, so daß die Möglichkeit besteht, Endoskop-Optiken nach dem Stand der Technik hiermit zu versehen. Es handelt sich also um ein Verfahren zur Beschichtung bekannter Art, dem entweder das Außen- oder das Innenrohr unterworfen wird. Verfahren zur Kunststoff-Beschichtung von Metallrohren sind bekannt und müssen deshalb dem Fachmann nicht im einzelnen erläutert werden.

- 6 -

Weiter ist bevorzugt, daß das Außenrohr 2 aus Metall und das Innenrohr 1 aus einem wärmedämmenden Kunststoff gefertigtwird. Dadurch wirkt das Außenrohr 2 gewissermaßen als Wärmeschild, dessen übergroße Wärme aber auf das Innenrohr 1 nicht vollständig übertragen werden kann, weil die Wärme durch das Kunststoff-Material zum größten Teil absorbiert wird. Auf diese Weise wird die direkte Wärmeleitung, wenn auch nicht verhindert, so doch entscheidend gehemmt. Es kommt hier nicht darauf an, die Wärmeleitung vollständig zu unterbinden, sondern es wird durch die Erfindung nur die übermäßige Erwärmung der Glasteile und der Kittstellen, mit denen diese im Innenrohr befestigt sind, verhindert.

Fig. 2 zeigt, daß zwischen dem Außenrohr 2 und dem Innenrohr 1 eine zusätzliche sehr dünne/Micro Schicht 11 oder ein zusätzliches sehr dünnes Rohr 11 aus Kunststoff-Folie angeordnet ist. Dies erscheint auch bei Endoskop-Optiken nach dem Stand der Technik möglich, bei denen das Außen- und das Innenrohr aus Metall gefertigt sind. Darüberhinaus ist aber bevorzugt, das Innenrohr 1, wie in der Fig. 1 , aus Kunststoff-Material zu fertigen. Gemäß der Fig. 2 kann das Rohr 1 jedoch wesentlich dünner gehalten sein als in der Fig. 1, weil durch das zusätzliche Rohr 11 eine zusätzliche Wärmedämmung eintritt.

00292602

- 7 -

Dadurch tritt insgesamt durch die Ausführungsform nach der Fig. 2 der Vorteil ein, daß trotz der Vergrößerung der Zahl der Rohre um das Rohr oder die Schicht 11 der Gesamtdurchmesser etwas kleiner gehalten werden kann als bei der Ausführungsform nach der Fig. 1.

Die Erfindung ist nicht auf die dargestellten Ausführungsformen beschränkt. Dem Fachmann ist vielmehr möglich, zusätzliche Ausführungsformen im Rahmen der Ansprüche zu erstellen.

00292602

## Ansprüche

1. Endoskop-Optik mit einem Außenrohr und einem die optischen Teile aus Glasmaterial wie Linsen oder Prismen aufnehmenden Innenrohr, dadurch gekennzeichnet, daß das Außenrohr (2) und das Innenrohr (1) aus unterschiedlichem Material bestehen, wobei zumindest eines ein wärmedämmendes Kunststoff-Material aufweist.

2. Endoskop-Optik nach Anspruch 1, dadurch gekennzeichnet, daß im Bereich der Stellen, wo optische Teile aus Glasmaterial (4-7) angeordnet sind, ein Spiel zwischen dem Außenrohr (2) und dem Innenrohr (1) vorgesehen ist.

3. Endoskop-Optik nach Anspruch 1, dadurch gekennzeichnet, daß das Außenrohr (2) aus Metall und das Innenrohr (1) aus einem wärmedämmenden Kunststoff gefertigt wird.

4. Verfahren zur Herstellung einer Endoskop-Optik nach Anspruch 1, dadurch gekennzeichnet, daß ein wärmedämmender Kunststoffbelag auf das Außenrohr (2) oder das Innenrohr(1) aufgebracht wird.

5. Endoskop-Optik nach Anspruch 1, dadurch gekennzeichnet,daß zwischen dem Außenrohr (2) und dem Innenrohr (1) ein zusätzli= ches sehr dünnes Rohr (11) aus Kunststoffolie angeordnet ist.

FIG.1

FIG. 2

1/1

00292602

Europäisches
Patentamt

**EUROPÄISCHER RECHERCHENBERICHT**

00292602

Nummer der Anmeldung

EP 87 11 1516

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 029 281 (OLYMPUS OPTICAL CO.,LTD.) * Zusammenfassung; Seite 1, Zeile 26 - Seite 2, Zeile 10; Figuren 1-4 * | 1,3-5 | A 61 B 1/00 |
| | --- | | |
| A | US-A-4 148 550 (R.B. MACANALLY) * Spalte 3, Zeile 67 - Spalte 4, Zeile 37, Figuren 1,2,4 * | 2 | |
| | --- | | |
| A | FR-A-1 347 596 (K. STORZ) * Seite 2, linke Spalte, Zeilen 27-40; Figuren 1-3 * | 1 | |
| | ----- | | |

|  |
|---|
| **RECHERCHIERTE SACHGEBIETE (Int. Cl.4)** |
| A 61 B 1/00 G 02 B 23/00 |

Der vorliegende Recherchenbericht wurde für alle Patentansprüche erstellt

| Recherchenort | Abschlußdatum der Recherche | Prüfer |
|---|---|---|
| BERLIN | 22-08-1988 | WEIHS J.A. |